# EUROPEAN PATENT APPLICATION

(11) **EP 2 554 159 A1**
(43) Date of publication of application: **06.02.2013**
(21) Application number: 11006436.7
(22) Date of filing: 04.08.2011
(51) Int. Cl.: A61K 9/00, A61K 9/16, A61K 9/28, A61P 7/02

(54) **Dosage forms comprising apixaban and content uniformity enhancer**

(71) Applicant: ratiopharm GmbH, 89079 Ulm (DE)
(72) Inventor: Stefan, Ralph, 88370 Ebenweiler (DE); Leutner, Dirk, 81371 München (DE)
(74) Representative: Aechter, Bernd

(57) **Abstract**

The invention relates to compositions and/or oral dosage forms comprising micronized apixaban and a content uniformity enhancer. The invention also relates to methods of preparing said dosage forms.

## Description

The invention relates to compositions and/or oral dosage forms comprising micronized apixaban and a content uniformity enhancer. The invention also relates to methods of preparing said dosage forms.

Apixaban is a low molecular selective inhibitor of the enzyme Faktor Xa, participating in the blood coagulation system. Apixaban is classified as an antithrombotic drug, which can be administered orally. Therefore, its possible medical indications are reported to be thrombosis treatment and thrombosis prophylaxis after orthopedic operations as well as the prophylaxis of ischaemic apoplexia in case of atrial fibrillation, the prophylaxis of the acute coronary syndrome and the prophylaxis after thrombosis and pulmonary embolism.

The IUPAC name for apixaban is [INN] 1-(4-Methoxyphenyl)-7-oxo-6- [4-(2-oxopiperidin-1-yl)phenyl]-4,5,6,7-tetrahydro-1*H*-pyrazolo[3,4-c]pyridin-3-carbamid. The chemical structure of apixaban is shown in the formula (1) below:

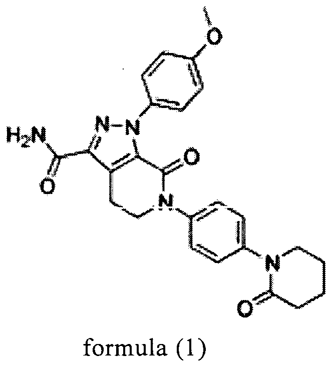

The synthesis of apixaban is described in WO 2003/026652 A1 by Bristol-Myers Squibb Company.

In WO 2006/078331 A2 and WO 2007/001385 A2 two polymorphous forms of apixaban are described. The H2-2 form is reported to be a needle-shaped dihydrate with a particle size D₉₀ greater than 160 µm. Further the H2-2 form is reported to be less stable than the granular, non-solvate N-1 form with a particle size D₉₀ smaller than 20 µm.

WO 2010/147978 A1 describes formulations comprising apixaban reportedly providing a controlled release of the API. Further, for comparison reasons, an immediate release formulation comprising crystalline Apixaban prepared by dry granulation is disclosed.

However, it turned out that prior art pharmaceutical formulations comprising apixaban and being suitable for oral administration are still improvable with regard to solubility and bioavailabilty. Further, stability and content uniformity of the known formulations may be improvable.

Hence, it was an object of the present invention to overcome the above-mentioned disadvantages.

In particular, it was an object of the invention to provide the active agent in a form, which possesses superior dissolution rates and bioavailability as well as superior storage stability and content uniformity. The above objects should be achievable without the use of a dry-granulation step. Further, it was the intention to provide the active agent in a form, which possesses good flowability and enables easy processing.

While developing apixaban formulations, the inventors of the present application were also confronted with the fact that crystalline apixaban can exist in different polymorphous forms, which have different solubility profiles and may tend to change into different polymorphous forms. In a patient, the different solubility profile leads to an undesirable, uneven rise in the concentration of the active agent. It was therefore an object of the present invention to provide apixaban in a form allowing a rise in the concentration in the patient as even as possible. The aim was largely to avoid both inter-individual and also intra-individual deviations.

### Summary of the invention

According to the present invention, the above objectives are achieved by a composition containing micronized apixaban and a content uniformity enhancer, which can be further processed to a dosage form, preferably by direct compression.

It was found that the composition and/or dosage of the present invention has superior dissolution rates and superior permeability, resulting in an excellent bioavailability of the active pharmaceutical ingredient. Further, superior content uniformity was achieved, even when dry-granulation was avoided, which ensured that the appropriate dose was applied to the patient. Even further it was found that the composition and/or dosage form of the present invention was very stable over a prolonged period of time. This is important, since apixaban is applied in low doses, and even little degradation of the drug can lower its beneficial effects.

A further subject of the invention concerns dosage forms comprising micronized apixaban and a content uniformity enhancer in the form of a composition.

Another subject of the invention is a method for producing a dosage form containing the composition of the invention comprising the steps of
(r1) providing a composition according to the invention,
(r2) mixing the composition from step (r1) with one or more pharmaceutical excipient(s), and
(r3) processing the mixture resulting from step (r2) into a dosage form, and
(r4) optionally film-coating the dosage form.

Finally, another subject of the invention is a dosage form, according to the invention, for the prevention or treatment of thrombotic disorders, wherein the dosage form is administered to a patient suffering from diabetes mellitus.

### Detailed description of the invention

In the context of this invention, the term "apixaban" comprises 1-(4-methoxyphenyl)-7-oxo-6- [4-(2-oxopiperidin-1-yl)phenyl]-4,5,6,7-tetrahydro-1H pyrazolo[3,4-c]pyridin-3-carbamid in accordance with formula (1) above. In addition, the term "apixaban" comprises all the pharmaceutically acceptable salts, hydrates and/or solvates thereof, for example apixaban hydrochloride. Preferably, apixaban is used in form of the free base, more preferably in the H2-2 form. Within the application, ratios or amounts of apixaban generally refer to the ratio or amount of apixaban in form of the free base, preferably without water of hydration. Further, the term "H2-2" form refers to the polymorphous form as described in WO 2007/001385 as "H2-2", in particular having characteristic diffraction peaks as described in section [00114] of WO 2007/001385.

For all the embodiments of this invention, the term "apixaban" preferably means apixaban in crystalline form, i.e. preferably more than 90 % by weight of the apixaban used is in crystalline form, especially 100 %. Further, in a preferred embodiment the crystalline apixaban which is to be micronized can preferably be present in the H2-2 form.

The expression "micronized apixaban" is used in the context of this invention to designate particulate apixaban, which generally has an average particle diameter (D₅₀-value) of 0.1 to 20 µm, preferably 0.3 to 10 µm, more preferably 0.5 to 5 µm and especially 1.0 to 4.0 µm.

The expression "average particle diameter" relates in the context of this invention to the D₅₀value of the volume-average particle diameter determined by means of laser diffractometry. In particular, a Malvern Instruments Mastersizer 2000 was used to determine the diameter (wet measurement, 2,000 rpm, ultrasound 60 sec., preferably shading 4 to 13 %, preferably dispersion in liquid paraffin, evaluated according to the Fraunhofer method). The average particle diameter, which is also referred to as the D₅₀-value of the integral volume distribution, is defined in the context of this invention as the particle diameter, at which 50 % by volume of the particles have a smaller diameter than the diameter which corresponds to the D₅₀ value. Similarly, 50 % by volume of the particles then have a larger diameter than the D₅₀ value.

Analogously, the D₁₀-value of the integral volume distribution is defined as the particle diameter at which 10 % by volume of the particles have a smaller diameter than the diameter which corresponds to the D₁₀-value. In a preferred embodiment the D₁₀-value of the integral volume distribution is from 0.01 to 7 µm, preferably from 0.05 to 5 µm, more preferably from 0.1 to 3.5 µm and especially from 0.5 to 1.5 µm.

Analogously, the D₉₀-value of the integral volume distribution is defined as the particle diameter at which 90 % by volume of the particles have a smaller diameter than the diameter which corresponds to the D₉₀-value. In a preferred embodiment the D₉₀-value of the integral volume distribution is from 0.5 to 30 µm, preferably from 1 to 15 µm, more preferably from 2.5 to 10 µm and especially from 3 to 8.5 µm.

Micronized apixaban in accordance with the invention is usually obtainable by milling.

The milling is generally performed in conventional milling apparatuses, such as in a ball mill, air jet mill, pin mill, classifier mill, cross beater mill, disk mill, mortar grinder, rotor mill. An air jet mill is preferably used.

The milling time is usually 0.5 minutes to 10 hours, preferably 2 minutes to 5 hours, more preferably 5 minutes to 3 hours.

In a preferred embodiment the ratio of the D₉₀-value to the D₁₀-value of the micronized apixaban is between 1 : 1 and 20 :1, more preferably between 1.1:1 and 12 :1 , even more preferably between 1.5 :1 and 9 : 1 and especially between 2:1 and 5 : 1. It appears that a small ratio of the D₉₀-value to the D₁₀-value seems to be favourable for good distribution of the active agent within the composition and therefore assures that the above mentioned objects are achieved.

In a preferred embodiment the integral volume distribution of apixaban can be monomodal or bimodal, preferably monomodal. That means that the integral volume distribution of apixaban shows only one maximum in a graph or histogram representing a probability distribution of the particle sizes.

In a preferred embodiment, the apixaban of the invention is present in micronized and stabilized form, namely in the form of a composition containing micronized apixaban and a content uniformity enhancer. In particular, the composition of the invention consists substantially of micronized apixaban and content uniformity enhancer. The expression "substantially" in this case indicates that small amounts of solvent etc. may also be present where applicable.

The content uniformity enhancer in the context of this invention can generally be a substance being capable of adsorbing to apixaban (chemically or physically). The content uniformity enhancer further can improve the stability and/or dissolution of the active pharmaceutical ingredient. Preferably, the content uniformity enhancer is capable of reducing the dissolution time of a pharmaceutical composition by 5 %, more preferably by 20 %, according to USP 31-NF26 release method, using apparatus II (paddle), compared to the same pharmaceutical composition comprising no content uniformity enhancer.

The composition of the invention may, for example comprise the following substances as content uniformity enhancer: cellulose derivates, such as hydroxypropyl methyl cellulose (HPMC), methyl cellulose, ethyl cellulose, hydroxyethyl cellulose, ethyl hydroxyethyl cellulose, hydroxypropyl cellulose (HPC), silicified microcrystalline cellulose, starch, starch derivatives, treated starch, chitin, maltodextrin, disaccharides, such as glucose, fructose, maltose, sucrose, sugar alcohols, such as isomalt, mannitol, sorbitol, xylitol and inorganic substances, such as silica, silicates and phosphates, Further, in principle, mixtures of the substrates mentioned are also possible.

Preferred substances used as content uniformity enhancer as matrix former are hydroxypropyl methyl cellulose (HPMC), methyl cellulose, ethyl cellulose,, silicified microcrystalline cellulose, mannitol, sucrose and inorganic substances, such as silica, silicates and phosphates. From these substances silicified microcrystalline cellulose, microcrystalline cellulose, silica, silicates - preferably aluminium magnesium silicates, such as Al₂O₃·MgO·1.7SiO₂·xH₂O - phosphates - preferably monocalcium phosphates, dicalcium phosphates and tricalcium phosphates - and mixtures thereof are especially preferred.

In a preferred embodiment the composition does not contain lactose, in particular anhydrous lactose, and/or microcrystalline cellulose.

In a preferred embodiment, the composition of the invention contains apixaban (or a pharmaceutically acceptable salt thereof) and content uniformity enhancer, the weight ratio of apixaban to content uniformity being 5 : 1 to 1 : 150, more preferably 1 : 1 to 1 : 50, even more preferably 1 : 2 to 1 :25, especially 1 : 5 to 1 : 15.

In a further preferred embodiment, the content uniformity enhancer can be a brittle compound. Pharmaceutical excipients can generally be classified with regard to the change in the shape of the particles under compression pressure (compaction): plastic excipients are characterised by plastic deformation, whereas when compressive force is exerted on brittle excipients, the particles tend to break into smaller particles. Brittle behaviour on the part of the excipients can be quantified by the increase in the surface area in a moulding. In the art, it is customary to classify the brittleness in terms of the "yield pressure". According to a possible classification, the values for the "yield pressure" here are low for plastic substances but high in case of friable substances on the other hand (Duberg, M., Nyström, C., 1982, "Studies on direct compression of tablets VI. Evaluation of methods for the estimation of particle fragmentation during compaction.", Acta Pharm. Suec. 19, 421-436; Humbert-Droz P., Mordier D., Doelker E., "Méthode rapide de détermination du comportement à la compression pour des études de préformulation", Pharm. Acta Helv., 57, 136-143 (1982)). The "yield pressure" describes the pressure that has to be reached for the excipient to begin to flow plastically.

The "yield pressure" is preferably calculated using the reciprocal of the gradient of the Heckel plot, as described in York, P., Drug Dev. Ind. Pharm. 18, 677 (1992). The measurement in this case is preferably made at 25 °C and at a deformation rate of 0.1 mm/s.

In the context of the present invention, an excipient (especially the content uniformity enhancer) is deemed a non-brittle excipient if it has a "yield pressure" of no more than 120 MPa, preferably no more than 100 MPa, particularly preferably 5 to 80 MPa. An excipient is usually described as a brittle excipient if it has a "yield pressure" of more than 80 MPa, preferably more than 100 MPa, particularly preferably more than 120 MPa, especially more than 150 MPa. Brittle excipients may exhibit a "yield pressure" of up to 300 MPa or up to 400 MPa or even up to 500 MPa.

Examples of brittle content uniformity enhancers are silicified microcrystalline cellulose, calcium phosphates, silicates or aluminosilicates. Especially preferred are silica, for example Aerosil^{®} 200, dicalcium phosphate (Dicafos AN) or magnesium aluminosilicate, for example, Al₂O₃·MgO·1.7SiO₂·xH₂O (Neusilin^{®}).

In a particular preferred embodiment the content uniformity enhancer generally has a specific surface area of 3.0 to 450 m²/g, more preferably 4.0 to 350 m²/g. Further, in a particular preferred embodiment the content uniformity enhancer generally has a specific surface area of 3.0 to 10 m²/g, preferably 5 to 8 m²/g. Still further, in an alternative particularly preferred embodiment the content uniformity enhancer has a specific surface area of 50 to 450 m²/g, more preferably 150 to 350 m²/g. The specific surface area preferably is determined by gas adsorption according Ph.Eur., 6th edition, Chapter 2.9.26. For this purpose an ASAP 2020 (Micromeritics) and an 'outgassing' temperature of 40 °C is used. It has surprisingly been found that the above-mentioned specific surface area can be beneficial in solving the above-mentioned objects, in particular, since substances with a high specific surface area are reported to reduce the bioavailability of apixaban.

Furthermore, a mixture of content uniformity enhancers is preferred, comprising a first content uniformity enhancer having a "high" specific surface area and a second content uniformity enhancer, having a "low" specific surface area. "High" refers to a specific surface area of 3.0 to 400 m²/g, more preferably 5 to 250 m²/g, still more preferably 15 to 200 m²/g, particularly 25 to 150 m²/g. "Low" refers to a specific surface area of 0.01 to 2.9 m²/g, more preferably 0.05 to 1.5 m²/g, still more preferably 0.1 to 1.5 m²/g, particularly 0.2 to 1.2 m²/g. Preferably, the weight ratio of enhancer with high surface area to enhancer with low surface area ranges from 1 : 10 to 10 : 1, more preferably from 5 : 1 to 1 : 5.

Examples of substances as content uniformity enhancer with an above-mentioned specific surface are silicified microcrystalline cellulose, fumed silica, such as Aerosil^{®}, and silicates, preferably aluminium magnesium silicates, such as Al₂O_{3·}MgO·1.7SiO₂·xH₂O.

It appears that a brittle content uniformity enhancer, in particular with the above specific surface area, can be advantageously used to solve the above-mentioned objects, in particular to provide a dosage form having superior content uniformity.

Further, in a preferred embodiment the content uniformity enhancer can be an inorganic substance. Examples of such inorganic substances are silica, silicates, phosphates, carbonates, sulfates and halogens with the corresponding counter ions, such as alkali, earth alkali, aluminium and boron. Possible inorganic content uniformity enhancers are phosphates, such as monocalcium phosphate, dicalcium phosphate and tricalcium phosphate, carbonates, such as magnesium carbonate and calcium carbonate and sulfates, such as calcium sulfate.

In a preferred embodiment of the invention the composition of the invention is obtained by jointly micronizing apixaban and the content uniformity enhancer. For this purpose, the non-micronized apixaban, preferably of the crystalline H2-2 form, and the content uniformity enhancer can preferably be mixed in a first step and the mixture is preferably milled in a second step. In alternative embodiments, the mixing may take place before or even during the milling, i.e. first step and second step may be performed simultaneously.

The process conditions in this embodiment are preferably selected such that the resulting composition particles have a volume-average particle diameter (D₅₀) of 1.0 to 90 µm, preferably 2 to 60 µm, more preferably 3 to 40 µm and especially 5 to 30 µm. Further, it is preferred that the integral volume distribution of the composition can be monomodal or bimodal, preferably monomodal.

Usually, micronisation of an active pharmaceutical ingredient (API) can entail disadvantages. For example, micronisation may result in an active agent with undesirably poor flowability. In addition, higher toxicity may make the micronised active ingredient more difficult and complicated to handle from the point of view of health and safety. The considerable enlargement of the surface area during micronisation may also cause the sensitivity of the active agent to oxidation to increase. However, in the present invention it has been surprisingly found that the composition of the present invention comprising micronized apixaban and content uniformity enhancer does not suffer from said expected drawbacks, however unexpectedly solves the above-mentioned objects.

Generally, the "composition" of the present invention can be used as intermediate or as final dosage form. Preferably, the composition of the present invention is used as intermediate, which is converted into the applicable dosage form of the present invention, usually by means of suitable methods, such as filling into sachets or capsules or by compressing into tablets. Optionally, before the filling or compression step the composition can be granulated.

Therefore, the composition of the invention can be employed to prepare a dosage form, preferably an oral dosage form, more preferably a solid oral dosage form, in particular a capsule or tablet, especially a tablet.

A preferred subject-matter of the invention is therefore a dosage form containing the composition of the invention and preferably one or more pharmaceutical excipients, preferably pharmaceutical excipients as described in the European Pharmacopoeia (Ph.Eur.) and/or in the US Pharmacopoeia (USP). In this regard it is noted that all explanations given above for the composition (e.g. relating to properties of apixaban and content uniformity enhancer) also apply to the dosage form of the present invention.

Examples of excipients used are disintegrants, binders, emulsifiers, anti-sticking agents, additives for improving the powder flowability and/or lubricants.

Disintegrants (c1) are reported to be substances, which accelerate the disintegration of a dosage form, especially a tablet, after having been placed into water. Suitable disintegrants are, for example, organic disintegrants, such as carrageenan, croscarmellose sodium, sodium carboxymethyl starch and crospovidone. Crospovidone and/or croscarmellose sodium are particularly preferred.

Alternatively, inorganic alkaline disintegrants are used, preferably salts of alkali metals and alkaline metals. Preferred alkali and alkaline metals are sodium, potassium, magnesium and calcium. As anions, carbonate, hydrogen carbonate, phosphate, hydrogen phosphate and dihydrogen phosphate are preferred. The term "alkaline disintegrants" means disintegrants which, when dissolved in water, produce a pH level of more than 7.0. Examples for inorganic alkaline disintegrants are sodium hydrogen carbonate, sodium hydrogen phosphate and calcium hydrogen carbonate.

Disintegrants can be present in an amount of 0 to 50 wt.%, preferably 5 to 40 wt.%, more preferably 7 to 30 wt.% and still more preferably 10 to 25 wt.% of the dosage form.

Binders usually are regarded as substances for ensuring that the oral dosage form (in particular the tablet) can be formed with the required mechanical strength.

The binder can be present in an amount of 0 to 40 wt.%, preferably 1 to 35 wt.%, more preferably 5 to 30 wt.% and still more preferably 7 to 20 wt.% of the dosage form.

The binder for the preparation of the intermediate of the dosage form is preferably a polymer. The polymer that can be used for the preparation of the dosage form preferably has a glass transition temperature (Tg) of more than 20 °C, more preferably 30 °C to 150 °C, especially 40 °C to 100 °C. The glass transition temperature is determined in the context of this invention by means of dynamic differential scanning calorimetry (DSC). For this purpose a Mettler Toledo DSC 1 apparatus can be used. The work is performed at a heating rate of 1-20 °C/min., preferably 5-15 °C/min., and at a cooling rate of 5-25 °C, preferably 10-20 °C/min.

The dosage form of the invention may, for example, comprise the following hydrophilic polymers as binder: polysaccharides, such as hydroxypropyl methyl cellulose (HPMC), methyl cellulose, hydroxyethyl cellulose, ethyl hydroxyethyl cellulose, hydroxypropyl cellulose (HPC); polyvinyl pyrrolidone, polyvinyl acetate (PVAC), polyvinyl alcohol (PVA), polymers of acrylic acid and their salts, polyacrylamide, polymethacrylates, vinyl pyrrolidone/vinyl acetate copolymers (such as Kollidon^{®} VA64, BASF), polyalkylene glycols, such as polypropylene glycol or preferably polyethylene glycol, co-block polymers of polyethylene glycol, especially co-block polymers of polyethylene glycol and polypropylene glycol (Pluronic^{®}, BASF), and mixtures of the polymers mentioned.

Substances particularly preferably used as binder are polyvinyl pyrrolidone, preferably with a weight average molecular weight of 10,000 to 60,000 g/mol, especially 12,000 to 40,000 g/mol, a copolymer of vinyl pyrrolidone and vinyl acetate, especially with a weight average molecular weight of 40,000 to 70,000 g/mol and/or polyethylene glycol, especially with a weight average molecular weight of 2,000 to 10,000 g/mol, and HPMC, especially with a weight average molecular weight of 20,000 to 90,000 g/mol and/or preferably a content of methyl groups of 10 to 35 % and a content of hydroxy groups of 1 to 35 %.

Co-block polymers of polyethylene glycol and polypropylene glycol can likewise preferably be used as binder, i.e. polyoxy-ethylene/polyoxypropylene block polymers. These polymers preferably have a weight average molecular weight of 1,000 to 20,000 g/mol, more preferably 1,500 to 12,500 g/mol, especially 5,000 to 10,000 g/mol. These block polymers are preferably obtained by condensation of propylene oxide with propylene glycol and subsequent condensation of the polymer formed with ethylene oxide. This means that the ethylene oxide content is preferably present as an "endblock". The block polymers preferably have a weight ratio of propylene oxide to ethylene oxide of 50 : 50 to 95 : 5, more preferably of 70 : 30 to 90 : 10. The block polymers preferably have a viscosity at 25 °C of 200 to 2,000 mPas, more preferably 500 to 1,500 mPas, especially 800 to 1,200 mPas.

The oral dosage form of the present invention may comprise emulsifiers. Possible emulsifiers are anionic emulsifiers, for example soaps, preferably alkali salts of higher fatty acids salts of bile acid (alkali salts); cation-active emulsifiers, for example benzalconium chloride, cetyl pyridinium chloride, cetrimide; non-ionic emulsifiers, e.g. sorbitan derivatives, especially sorbitan monolaurate, polyoxythylene-(20)-sorbitan-monolaurate, polyethylene glycol derivatives/polyoxyethylene derivatives, especially polyoxyethylene-(20)-sorbitan monostearate, polyoxyethylene stearate or polyoxyethylene stearyl ether. In addition, partial fatty acid esters of polyhydric alcohols can be used, such as glycerol monostearate, fatty acid ester of sucrose, fatty acid ester of polyglycol or casein. Furthermore, mixtures of the above-mentioned substances can be used.

The emulsifier may be present in an amount of 0 to 10 wt.%, preferably in an amount of 0.5 to 7 wt.% of the dosage form.

The oral dosage form of the present invention may comprise anti-sticking agents. Anti-sticking agents are usually understood to mean substances reducing agglomeration in the core bed. Examples are talcum, silica gel, polyethylene glycol (preferably with 2,000 to 10,000 g/mol weight average molecular weight) and/or glycerol monostearate. Examples of preferred anti-sticking agents are talcum and polyethylene glycol (Mg 3,000-6,000 g/mol) and carrageenan. Anti-sticking agents may be present in an amount of 0 to 5 wt.%, more preferably in an amount of 0.5 to 3 wt.% of the dosage form.

An example of an additive to improve powder flowability is disperse or colloidal, silica (for example Aerosil^{®}). Additives to improve powder flowability may be present in an amount of 0 to 5 wt.%, preferably 1 to 4 wt.% of the total weight of the dosage form.

In addition, lubricants may be used. Lubricants are generally used in order to reduce sliding friction. In particular, the intention is to reduce the sliding friction found during tablet pressing between the punch moving up and down in the die and the die wall, on the one hand, and between the edge of the tablet and the die wall, on the other hand. Suitable lubricants are, for example, stearic acid, adipic acid, sodium stearyl fumarate and/or magnesium stearate.

Lubricants are generally used in an amount of 0.1 to 3 % by weight, based on the total weight of the dosage from.

The ratio of active agent to excipients is preferably selected such that the resulting dosage form contain 0.1 to 10 % by weight, more preferably 1.5 to 6 % by weight, especially 2 to 4 % by weight apixaban, preferably micronized apixaban and 90 to 99 % by weight, more preferably 94 to 98.5 % by weight, especially 96 to 98% by weight pharmaceutically acceptable excipients.

Content uniformity enhancers are normally used in an amount of 10 to 99 % by weight, preferably 20 to 90 % by weight, more preferably 25 to 80 %, in particular 40 to 75 % by weight, based on the total weight of the dosage form.

In these ratios specified, the amount of content uniformity enhancer used to prepare the composition of the invention is counted as an excipient. This means that the amount of active agent refers to the amount of micronized apixaban contained in the dosage form. Preferably, the dosage form comprises 0.1 to 50 mg apixaban, more preferably 0.5 to 20 mg, in particular 1 to 10 mg.

In a preferred embodiment the oral dosage form of the present invention comprises
0.1 to 10 %, preferably 1.5 to 6 % by weight apixaban,
15 to 90 %, preferably 30 to 80 % by weight content uniformity enhancer,
1 to 40 %, preferably 5 to 25 % by weight binder,
1 to 30 %, preferably 5 to 20 % by weight disintegrant and optionally
0.1 to 5 %, preferably 0.5 to 3 % by weight lubricant.

It lies in the nature of pharmaceutical excipients that they sometimes perform more than one function in a pharmaceutical formulation. In the context of this invention, in order to provide an unambiguous delimitation, the fiction will therefore preferably apply that a substance which is used as a particular excipient is not simultaneously also used as a further pharmaceutical excipient. For example, silica - if used as a content uniformity enhancer - is not additionally used as an additive to improve powder flowability (even though silica also improves powder flowability). Similarly, microcrystalline cellulose - if used as a content uniformity enhancer - is not additionally used as a disintegrant, (even though microcrystalline cellulose also exhibits a certain disintegrating effect).

In a preferred embodiment the dosage form according to the invention provides an immediate release (IR) of apixaban. This means that the release profile of the dosage forms of the invention according to USP method (paddle, 900ml, 0.1 n HCl, 75 rpm, 37°C) after 10 minutes usually indicates a content release of at least 75 %, preferably at least 85 %, especially at least 90 %.

In a preferred embodiment the dosage form of the present application can be a tablet.

The tablet of the invention preferably can have a hardness of 25 o to 250 N, particularly preferably of 30 to 180 N or 40 to 150 N. The hardness is determined in accordance with Ph.Eur., 6.0, Chapter 2.9.8.

In addition, the tablet of the invention preferably can have a friability of less than 3 %, more preferably less than 2 %, in particular 0.1 to 1.2 %. The friability is determined in accordance with Ph.Eur., 6.0, Chapter 2.9.7.

Further, the tablet of the invention preferably can have a "content uniformity" of 93 to 107 %, more preferably 95 to 105 %, still more preferably 98 to 102 %, particularly 99 to 101 % of the average content. The "content uniformity" is determined in accordance with Ph.Eur., 6.0, Chapter 2.9.6.

The above details regarding hardness, friability and content uniformity preferably relate to the non-film-coated tablet. Further, the above-mentioned ratios of active agent to excipients relate to the uncoated tablet.

The dosage form of the invention tablets may be a tablet which can be swallowed unchewed (non-film-coated or preferably film-coated). It may likewise be a chewable tablet or a dispersible tablet. "Dispersible tablet" means a tablet to be used for producing an aqueous suspension for swallowing.

In a preferred embodiment, the tablet of the present application can be film-coated. For this purpose, methods known in the art for film-coating a tablet may be employed.

Generally, film-coatings can be prepared by using cellulose derivatives, poly(meth)-acrylate, polyvinyl pyrrolidone, polyvinyl acetate phthalate, and/or shellac or natural rubbers such as carrageenan.

In a preferred embodiment of the present invention the film-coating can be a film-coating, essentially without affecting the release of the active agent, or a film-coating affecting the release of the active agent.

Preferred examples of film-coatings, which do not affect the release of the active ingredient, can be those including poly(meth)acrylate, methylcellulose (MC), hydroxypropyl methylcellulose (HPMC), hydroxypropyl cellulose (HPC), hydroxyethyl cellulose (HEC), polyvinyl pyrrolidone (PVP) and mixtures thereof. These polymers can have an average molecular weight of 10,000 to 150,000 g/mol.

In an alternative preferred embodiment, the film-coating can affect the release of the active agent. Examples for film-coating affecting the release of the active agent are gastric juice resistant film-coatings and retard coatings.

In gastric juice resistant coatings the solubility depends on the pH of the surrounding.

Examples of gastric juice 'resistant' coatings can comprise cellulose acetate phthalate (CAP), hydroxypropyl methylcellulose phthalate (HPMCP and polyvinyl acetate phthalate (PVAP).

Retard coatings are usually non-soluble (preferably having a water solubility at 25 °C of less than 10 mg/ml).

Examples of retard coatings can comprise ethyl cellulose (EC, commercially available e.g. as Surelease^{®}) and poly(meth)acrylate (commercially available e.g. as Eudragit^{®} RL or RS and L/S).

Further the coating can be free from active ingredient. However, it is also possible that the coating can contain an active ingredient (apixaban). In such a case, this amount of active ingredient would function as an initial dose. In such a case, the coating preferably can comprise 1 to 45 wt.%, preferably 5 to 35 wt.%, most preferably 10 to 30 wt.% of apixaban, based on the total amount of apixaban contained in the tablet.

In case the film coating does not contain apixaban (which is preferred), it can have a thickness of 2 µm to 100 µm, preferably from 20 to 60 µm. In case of a coating containing apixaban, the thickness of the coating is usually 10 µm to 200 µm, preferably from 50 to 125 µm.

The preferred coating of the invention may comprise (e1) solubilizer; (e2) lubricant; (e3) surfactant; (e4) glidant; (e5) pigment; and/or (e6) water.

In an alternative preferred embodiment the solubility of the coating can be based on a pH-dependent binder. For this purpose, a poly(meth)acrylate polymer can be preferably contained as a solubilizer (e1). Such a preferred poly(meth)acrylate polymer can dissolve readily in acidic media of pH < 5 via salt formation and, therefore, does not affect further tablet disintegration and release behaviour. A preferred poly(meth)acrylate polymer (for example Eudragit^{®} E PO) can have the following chemical structure (2): 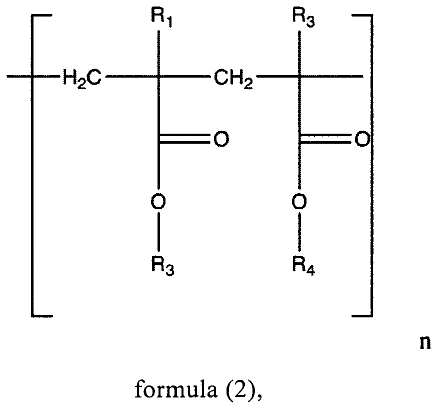 wherein R₁ and R₂ are CH₃, R₃ is (CH₂)₂N(CH₃)₂, and R₄ is CH₃ or C₄H₉. The average molecular weight is from 20000-300000 g/mol, more preferred from 30000-150000 g/mol and particularly from 35000-80000 g/mol.

The preferred coating according to an embodiment of the present invention can, along with the solubilizer (e1), comprise stearic acid as lubricant (e2) for plasticizing and dissolving the polymer, sodium lauryl sulfate as a surfactant (e3) for wetting and dispersing, talc as glidant (e4), iron oxide yellow and/or titanium oxide as pigment/s (e5) and, optionally, purified water.

A further subject of the invention is a method for preparing the dosage form of the present invention comprising the steps of
(r1) providing micronized apixaban and content uniformity enhancer,
(r2) mixing the composition from step (r1) with optionally one or more further excipients and
(r3) processing the mixture resulting from step (r2) into a dosage form, and,
(r4) optionally film-coating the dosage form.

In step (r1) a composition, containing micronized apixaban and a content uniformity enhancer, is provided. As mentioned above, the composition can be prepared by blending and/or jointly micronizing said compounds.

In a preferred embodiment, step (r2) can be characterized by mixing the composition of step (r1) with one or more excipient(s) as outlined above. The mixing (r2) can be carried out with conventional mixing devices. In order to ensure an even distribution, mixing in intensive mixers is preferable. Suitable mixing devices can preferably be compulsory mixers or free fall mixer, for example a Turbula^{®} T 10B (Bachofen AG, Switzerland). Mixing can be carried out, for example, for 1 minute to 1 hour, preferably for 5 to 30 minutes.

In an alternative embodiment, mixing (r2) can be conducted such that the composition of step (r1) can be mixed with a first part of the excipient(s) in a mixing device, for example in a high shear or tumbler mixer. After this first mixing step a second part of the excipient(s) can be added, which may be followed by a second mixing step. This procedure can be repeated until the last part of the excipient(s) is used, preferably one to five times. This kind of mixing can assure an even distribution of active agent and provide a mass for further processing in step (r3), for example for a tableting process.

Alternatively, steps (r1) and (r2) can be carried out together.

In step (r3) the mixture resulting from step (r2) can be further processed into a dosage form. For this purpose, said mixture can, for example, be filled into sachets or capsules.

In a preferred embodiment step (r3) can include compressing the mixture of step (r2) into tablets.

The compression of the mixture of step (r2) can be preferably a direct compression. This direct compression step can preferably be carried out on a rotary press, for example on a Fette^{®} 102i (Fette GmbH, Germany) or a Riva^{®} piccola (Riva, Argentina). If a rotary press is applied, the main compaction force can range from 1 to 50 kN, preferably from 2 to 40 kN, more preferably form 3 to 30 kN.

In step (r4) the tablet can preferably be film-coated, either with a film-coating affecting the release of the active agent or with a film-coating not affecting the release of the active agent.

It has, however, become apparent that the properties of the resulting tablets can be positively influenced when the dosage form of the invention is subjected to wet granulation step.

Hence, another preferred embodiment of the invention is the method of preparing a dosage form comprising the steps of
(s1) providing a composition comprising apixaban and content uniformity enhancer, and, optionally pharmaceutical excipients,
(s2) wetting or suspending the substances from step (s1) with or in a granulation liquid,
(s3) granulating the resulting mixture,
(s4) drying and, where appropriate, screening the granulates obtained,
(s5) processing the granulates resulting from step (s4) into a dosage form, and
(s6) optionally film-coating the dosage form.

In step (s1) apixaban, preferably micronized apixaban, and content uniformity enhancer according to the invention and, optionally, pharmaceutical excipients are provided. The pharmaceutical excipients can preferably be chosen from the excipients described above.

The substances are preferably mixed. In order to ensure an even distribution, mixing in intensive mixers is preferred. The mixing may, for example, be performed in compulsory mixers or free-fall mixers. Essentially, the explanations given above for step (r1) also apply here for step (s1).

The substances from step (s2) are wetted with a granulation liquid or suspended in a granulation liquid. The granulation liquid preferably comprises a binder (as illustrated above. Preferably, the granulation liquid containing binder is a solution or a suspension, preferably a solution. Suitable liquids for preparing the granulation liquid are, for example, water, alcohols and mixtures thereof. A mixture of water and ethanol is preferred.

Steps (s1) to (s4) can be carried out in standard granulation apparatuses. The "one-pot process" or the "fluidised-bed process" is preferred.

In the one-pot process, the substances from step (s1) are wetted and granulated with granulation liquid. Steps (s2) and (s3) are preferably performed concurrently. The granulates are then dried and optionally screened. A suitable granulating machine is, for example, Diosna^{®} P1/6.

In the fluidised-bed process, the substances from step (s1) are suspended in the granulation liquid and sprayed to dry them. The mixing, wetting, granulating and drying are performed in one operation. The granulates are then optionally screened. A suitable fluidised-bed granulator is, for example, a Glatt^{®} GPCG 3.

In a preferred embodiment, the granulation conditions are selected such that the resulting particles (granulates) have an average particle size (D₅₀) of 50 to 600 µm, more preferably 100 to 500 µm, even more preferably 150 to 400 µm, especially 200 to 350 µm.

In addition, the granulation conditions are preferably selected such that the resulting granulates have a bulk density of 0.2 to 0.85 g/ml, more preferably 0.3 to 0.8 g/ml, especially 0.4 to 0.7 g/ml. The Hausner factor is usually in the range from 1.03 to 1.3, more preferably from 1.04 to 1.20 and especially from 1.04 to 1.15. The "Hausner factor" in this context means the ratio of compacted density to bulk density.

The granulates resulting from step (s4) can be further processed into pharmaceutical dosage forms. For this purpose, the granulates are, for example, filled into sachets or capsules. The granulates resulting from step (s4) are preferably compressed into tablets. Regarding the compression step (s5), reference is made to the explanations given above for step (r3).

In step (s6) the tablet can preferably be film-coated, either with a film-coating affecting the release of the active agent or with a film-coating not affecting the release of the active agent.

The above-mentioned problems of the prior art can further be overcome by a melt-granulation or melt-extrusion process for the manufacture of a pharmaceutical formulation of apixaban. Hence, another embodiment of the invention is the method of preparing a dosage form comprising the steps of
(t1) mixing apixaban, preferably micronised apixaban, and excipients, perferably a content uniformity enhancer and/or a binder,
(t2) melting the mixture,
(t3) granulating the melted mixture,
(t4) mixing the granulates from step (t3) with optionally further excipients,
(t5) processing the mixture resulting from step (t4) into a dosage form,and
(t6) optionally film-coating the dosage form.

In a preferred embodiment the dosage form may be administered in a single daily dose or in divided doses two to six times a day. In certain embodiments of the invention the dosage form of apixaban may be administered less frequent then once daily, e.g. every second, third, or fourth day. In particular, the dosage form of the present invention is administered twice daily. It has been unexpectedly found that the dosage form of the present invention can be administered independently from the meals of the patient, i.e. the dosage forms of the present invention are suitable to be administered before, during or after the meals.

In a preferred embodiment, the dosage forms of the invention are administered daily at a dose of from about 0.1 to about 25 mg/d, preferably 1,0 to 10 mg/d, more preferably 2,5 to 5 mg/d.

The dosage form of the present invention preferably shows upon administration superior dissolution and absorption characteristics, leading to desirable plasma values like desirable AUC (area under the curve from 0 to 48 hours after oral administration), Cₘₐₓ and Tₘₐₓ values. Preferably, the administration of the dosage form of the present invention results in a Tₘₐₓ value of 1 to 5 hours, more preferably 2.0 to 3.5 hours. Preferably, administration of the dosage form of the present invention results in a Cₘₐₓ value of 20 to 200 ng/ml, more preferably 50 to 150 ng/ml. Preferably, administration of the dosage form of the present invention results in a AUC value of 150 to 4000 ng h/ml, more preferably 500 to 2500 ng h/ml. The plasma values are an average of ten single measurement values determined upon administration to 10 male humans having a body weight of about 75 kg.

The dosage forms of the present invention are used in the management (e.g. in the prevention or treatment) of thrombotic disorders. Hence, a further subject of the invention is a dosage form according to the invention for managing thrombotic disorders. It has been found, that patients having at least one of the following risks can be advantageously treated: age 66 years or older, elevated cardiac biomarkers, cerebrovascular diseases, peripheral vascular disease, non-revascularizable multivessel coronary artery disease, renal insufficiency and diabetes mellitus. Preferably, the dosage form according to the invention can be used for preventing or treating thrombotic disorders, wherein the dosage form is administered in a patient suffering from diabetes mellittus.

Hence, a further subject of the invention is a dosage form for the prevention or treatment of thrombotic disorders comprising an excipient having a specific surface area of 2.0 to 400 m²/g, wherein the dosage form has a content uniformity of 95 to 105 %. Alternatively, a subject of the invention is the use of an excipient having a specific surface area of 2.0 to 400 m²/g for the production of a dosage form for the prevention or treatment of thrombotic disorders, said dosage form having a content uniformity of 95 to 105 %. All explanations give above for preferred embodiments (apixaban as agent for the prevention or treatment of thrombotic disorders, preferred content uniformity enhancers, etc.) also apply to said subject.

### EXAMPLES

| **Example 1** | |
|---|---|
| Apixaban (calculated as free base without water of hydration) | 2.5 mg |
| Aluminium magnesium silicate (e.g. Neusilin^{®} US2) | 60 mg |
| Corn starch | 23.5 mg |
| Crospovidone (e.g. Kollidon^{®} CL) | 10 mg |
| Sodium dodecyl sulphate | 2 mg |
| Silicium dioxide, fumed, hydrophilic, highly dispersible | |
| (e.g. AEROSIL^{®} 200) | 1 mg |
| Magnesium stearate | 1 mg |
| **TOTAL** | **100 mg** |

Apixaban and aluminium magnesium silicate are sieved through mesh 1 mm and blended in a tumble mixer for 15 min. In a second step the other excipients, except magnesium stearate, are added after sieving (mesh 0.5 mm) and mixed for 10 min. Magnesium stearate is added and mixed for another 5 min before pressing into tablets with a hardness ranging about 50-150 N.

| **Example 2** | |
|---|---|
| Apixaban (calculated as free base without water of hydration) | 2.5 mg |
| Silicified microcrystalline cellulose (e.g. Prosolv SMCC 90) | 89 mg |
| Crospovidone (e.g. Kollidon^{®} CL) | 5 mg |
| Sodium dodecyl sulfate | 2 mg |
| Silicium dioxide, fumed, hydrophilic, highly dispersible | |
| (e.g. AEROSIL^{®} 200) | 0.5 mg |
| Magnesium stearate | 1 mg |
| **TOTAL** | **100 mg** |

Apixaban and silicified microcrystalline cellulose are sieved through mesh 1 mm and blended in a tumble mixer for 15 min. In a second step the other excipients except magnesium stearate are added after sieving (mesh 0.5 mm) and mixed for 10 min. Magnesium stearate is added and mixed for another 5 min before pressing into tablets with a hardness ranging about 50-150 N.

| **Example 3** | |
|---|---|
| Apixaban (calculated as free base without water of hydration) | 5 mg |
| Dicalciumphosphate, anhydrous (e.g. Dicafos AN) | 51.5 mg |
| Eudragit^{®} L 100-55 | 35 mg |
| Crospovidone (e.g. Kollidon^{®} CL) | 5 mg |
| Sodium dodecyl sulfate | 2 mg |
| Silicium dioxide, fumed, hydrophilic, highly dispersible | |
| (e.g. AEROSIL^{®} 200) | 0.5 mg |
| Magnesium stearate | 1 mg |
| **TOTAL** | **100 mg** |

Apixaban and anhydrous dicalciumphosphate are sieved through mesh 1 mm and blended in a tumble mixer for 10 min. The blend and all other excipients, excluding magnesium stearate are sieved (mesh 500 µm) and mixed together in a tumble mixer for 15 min. The premix is compacted and granulated through a sieve (mesh 800 µm). Magnesium stearate is added through a sieve (mesh 500 µm) and mixed together with the premix for further 5 min in a tumble blender to the final blend for compression. The final blend is compressed to tablets with a hardness of 50 - 150 N.

| **Example 4** | |
|---|---|
| Tablet core | |
| Apixaban (calculated as free base without water of hydration) | 5 mg |
| Dicalciumphosphate, anhydrous (e.g. Dicafos AN) | 59.5 mg |
| Sodium dodecyl sulfate | 2 mg |
| Povidone 30 (e.g. Kollidon^{®} 30) | 2 mg |
| silicium dioxide, fumed, hydrophilic, highly dispersible | |
| (e.g. AEROSIL^{®} 200) | 0.5 mg |
| Magnesium stearate | 1 mg |
| **TOTAL** | **70 mg** |

| Tablet coating: | |
|---|---|
| Tablet core | 70 mg |
| Eudragit^{®} NE30 D | 20 mg (calculated to polymer content) |
| Polysorbate 80 | 0.4 mg |
| Glycerol monostearate | 1.0 mg |

Apixaban and anhydrous dicalciumphosphate are sieved through mesh 1 mm and blended in a tumble mixer for 10 min. The preblend, SDS and Povidone 30 are sieved (mesh 1000 µm) and mixed together in a high shear mixer for 2 min. The premix is granulated with water until a "snowball" consistence is reached. The wet granules are sieved (mesh 2000 µm) and dried in a cabinet dryer at 40° C for 2 h. The dry granules are sieved (mesh 1250 µm) and the residual excipients are added through a sieve (mesh size # 800 µm) and mixed together with the granules for further 5 min in a tumble blender. The final blend is compressed on an eccentric press (Korsch EKO) tablets with a hardness of 50 - 150 N. The tablet core is coated with the prepared coating solution of Eudragit^{®} NE30D in a pan coater.

## Claims

1. A composition containing micronized apixaban and a content uniformity enhancer.

2. The composition according to claim 1, wherein apixaban has an average particle diameter (D₅₀-value) of 0.1 to 20 µm.

3. The composition according to claim 1 or 2, wherein the ratio of the D₉₀-value to D₁₀-value is between 1 : 1 and 20 : 1.

4. The composition according to any one of claims 1 to 3, wherein the weight ratio of apixaban to content uniformity enhancer is 1 : 1 to 1 : 50, preferably 1 : 2 to 1 : 15.

5. The composition according to any one of claims 1 to 4, wherein the content uniformity enhancer is a brittle compound with a yield pressure of at least 80 MPa.

6. The composition according to any one of claims 1 to 5, wherein the content uniformity enhancer has a specific surface area of 2.0 to 400 m²/g, determined by gas adsorption according Ph.Eur., 6^{th} edition, chapter 2.9.26.

7. The composition according to any one of claims 1 to 6, obtained by jointly micronizing apixaban and the content uniformity enhancer.

8. A dosage form containing a composition according to any one of claims 1 to 7 and, optionally, one or more pharmaceutical excipient(s).

9. A dosage form according to claim 8, wherein the dosage form provides immediate release of apixaban.

10. A dosage form according to claim 8 or 9, wherein the dosage form is in form of a tablet, said tablet having a hardness of 40 to 150 N, a friability of less than 2 % and/or a content uniformity of 95 to 105 %.

11. A dosage form according to claim 10, wherein the tablet is film-coated.

12. A dosage form according to claim 11, where the film-coating is a film-coating without affecting the release of the active agent or a film-coating affecting the release of the active agent.

13. Dosage form for the prevention or treatment of thrombotic disorders, comprising an excipient having a specific surface area of 2.0 to 400 m²/g, wherein the dosage form has a content uniformity of 95 to 105 %.

14. A dosage form according to claims 8 to 13 for the prevention or treatment of thrombotic disorders, wherein the dosage form is administered to a patient suffering from diabetes mellitus.

15. A method of preparing a dosage form according to any one of claims 8 to 14, comprising the steps of
(r1) providing a composition according to any one of claims 1 to 7,
(r2) mixing the composition from step (r1) with one nr more pharmaceutical excipient(s),
(r3) processing the mixture resulting from step (r2) into a dosage form, and
(r4) optionally film-coating the dosage form.
